# EUROPEAN PATENT SPECIFICATION

(11) **EP 2 567 234 B1**
(45) Date of publication and mention of the grant of the patent: **19.09.2018**
(21) Application number: 11723891.5
(22) Date of filing: 09.05.2011
(51) Int. Cl.: A61K 49/00, C07K 16/32, A61K 39/00, G01N 33/566, C07K 16/42, C07K 16/18, G01N 33/574, C07K 16/28

(54) **DIAGNOSTIC METHOD FOR THE DETECTION OF CELLS EX VIVO**
Diagnoseverfahren zum Nachweis von Zellen ex vivo
Procédé de diagnostic pour la détection de cellules ex vivo

(30) Priority: 07.05.2010 EP 10004892
(43) Date of publication of application: 13.03.2013
(73) Proprietor: F. Hoffmann-La Roche AG, 4070 Basel (CH)
(72) Inventor: DOBOSZ, Michael, 82393 Iffeldorf (DE); SCHEUER, Werner, Penzberg 82377 (DE)
(74) Representative: Schiweck Weinzierl Koch Patentanwälte Partnerschaft mbB
(86) International application number: PCT/EP2011/057400
(87) International publication number: WO 2011/138462

(56) References cited:
- WO-A2-2008/033495
- WO-A2-2008/148546
- DE DECKER M ET AL: "In vitro and in vivo evaluation of direct rhenium-188-labeled anti-CD52 monoclonal antibody alemtuzumab for radioimmunotherapy of B-cell chronic lymphocytic leukemia", NUCLEAR MEDICINE AND BIOLOGY, ELSEVIER, NY, US LNKD- DOI:10.1016/J.NUCMEDBIO.2008.03.001, vol. 35, no. 5, 1 July 2008 (2008-07-01), pages 599-604, XP022851421, ISSN: 0969-8051 [retrieved on 2008-06-25]
- DAHLE J ET AL: "Relative Biologic Effects of Low-Dose-Rate alpha-Emitting <227>Th-Rituximab and beta-Emitting <90>Y-Tiuexetan-Ibritumomab Versus External Beam X-Radiation", INTERNATIONAL JOURNAL OF RADIATION: ONCOLOGY BIOLOGY PHYSICS, PERGAMON PRESS, USA, vol. 72, no. 1, 1 September 2008 (2008-09-01), pages 186-192, XP025407376, ISSN: 0360-3016, DOI: 10.1016/J.IJROBP.2008.05.029 [retrieved on 2008-08-20]
- OUDE MUNNINK T H ET AL: "Zr-trastuzumab PET visualises HER2 downregulation by the HSP90 inhibitor NVP-AUY922 in a human tumour xenograft", EUROPEAN JOURNAL OF CANCER, PERGAMON PRESS, OXFORD, GB, vol. 46, no. 3, 1 February 2010 (2010-02-01), pages 678-684, XP026881622, ISSN: 0959-8049, DOI: 10.1016/J.EJCA.2009.12.009 [retrieved on 2010-01-30]
- MIKAEL PERSSON ET AL: "[177Lu]pertuzumab: experimental studies on targeting of HER-2 positive tumour cells", EUROPEAN JOURNAL OF NUCLEAR MEDICINE AND MOLECULAR IMAGING, SPRINGER, BERLIN, DE, vol. 32, no. 12, 1 December 2005 (2005-12-01), pages 1457-1462, XP019332775, ISSN: 1619-7089, DOI: 10.1007/S00259-005-1902-0
- J. H.E. BAKER ET AL: "Direct Visualization of Heterogeneous Extravascular Distribution of Trastuzumab in Human Epidermal Growth Factor Receptor Type 2 Overexpressing Xenografts", CLINICAL CANCER RESEARCH, vol. 14, no. 7, 1 April 2008 (2008-04-01), pages 2171-2179, XP55078696, ISSN: 1078-0432, DOI: 10.1158/1078-0432.CCR-07-4465
- KOYAMA YOSHINORI ET AL: "In vivo molecular imaging to diagnose and subtype tumors through receptor-targeted optically labeled monoclonal antibodies", NEOPLASIA, NEOPLASIA PRESS, ANN ARBOR, MI, US , vol. 9, no. 12 1 December 2007 (2007-12-01), pages 1021-1029, XP008140668, ISSN: 1522-8002 Retrieved from the Internet: URL:http://www.neoplasia.com/abstract.php? msid=1313 [retrieved on 2016-04-01]

## Description

The present invention relates in essence to a immunohistochemistry (IHC) method for detecting a subset of cells with a binding domain which is specific for a target which characterizes said subset of cells, which method comprises detecting in a tissue section, ex vivo, said subset of cells, wherein said binding domain is to be administered to a subject which comprises or is assumed to comprise said subset of cells, prior to the removal of said subset of cells, wherein said binding domain is labeled and wherein said subset of cells is detected by direct immunohistochemistry. The present invention also relates to the use of a binding domain as defined herein in a method defined herein. The use of a binding domain as defined herein for the preparation of a diagnostic composition to be used in a method defined herein is also envisaged. In another aspect, the present invention relates to the binding domain as defined herein to be used in a method defined herein. Kits comprising a binding domain as defined herein and means to administer said binding domain to a subject, and means to detect said binding domain with a method defined herein, are also disclosed. In another aspect, the present disclosure relates to the use of a binding domain, preferably a therapeutically effective antibody like for example alemtuzumab, apolizumab, cetuximab, epratuzumab, galiximab, gemtuzumab, ipilimumab, labetuzumab, panitumumab, rituximab, trastuzumab, nimotuzumab, mapatumumab, matuzumab, rhMab ICR62, rhMab B-Ly1 and pertuzumab etc including combinations thereof, for the preparation of a pharmaceutical composition for the treatment of patients disposed to respond favorably to said binding domain as identified by a method defined herein.

Targeting a tumor-associated surface antigen by a monoclonal antibody is a successful approach for the treatment of different malignancies. However, before therapeutic intervention with a given antibody is initiated, expression of the relevant antigen has to be confirmed. This is frequently accomplished by conventional immunohistochemistry (IHC) or in situ hybridization assays after formalin fixation and paraffin-embedding procedures of explanted tumor tissue. Treatment with a therapeutic antibody is only justified if expression of the relevant tumor-associated target antigen has been confirmed. Biodistribution methods based on radiolabelled antibodies have been discussed as well (Decker et al., Nucl. Med. And Biol., 35 (2008),pp. 599-604). However, these methods give merely some insight into the biodistribution of antibodies within the living body and/or explanted organs.

Immunohistochemistry (IHC) is the localization of antigens in tissue sections by the use of labeled antibody as specific reagents through antigen-antibody interactions that are visualized by a marker such as fluorescent dye, enzyme, radioactive element or colloidal gold. Immunohistochemistry generally consists of the following steps: a) a primary antibody binds to a specific antigen; b) the antibody-antigen complex is bound by a secondary, labeled, antibody; c) the label and thereby the antigen (if present) is detected at the sites of antibody-antigen binding. The second antibody is generally employed in order to enhance the detection signal which, otherwise might be to low for a sufficient detection.
Albert H. Coons and his colleagues (Coons et al. 1941, 1955; Coons and Kaplan 1950) were the first to label antibodies with a fluorescent dye, and used it to identify antigens in tissue sections. With the expansion and development of immunohistochemistry technique, enzyme labels have been introduced such as peroxidase and alkaline phosphatase, as well as colloidal gold to name some. Immunohistochemical staining is widely used in the diagnosis of abnormal cells such as those found in cancerous tumors. Other specific molecular markers are characteristic of particular cellular events such as proliferation or cell death. IHC is also widely used in basic research to understand the distribution and localization of biomarkers and differentially expressed proteins in different parts of a biological tissue. Since immunohistochemistry involves specific antigen-antibody reaction, it has apparent advantage over traditionally used special enzyme staining techniques that identify only a limited number of proteins, enzymes and tissue structures. Therefore, immunohistochemistry has become a crucial technique and widely used in many medical research laboratories as well as clinical diagnostics. WO2008/033495 reports on the surprising finding that a certain antibody clone which is specific for the EGF-receptor may be useful for the detection and therapy of skin diseases, in particular for psoriasis. Said antibody is also suggested for in vivo imaging methods and for a "classical" IHC based method. While the in vivo imaging method requires the administration of the antibody prior to the detection (see claims 26 and 33 as well as paragraph [0041] of said document), it is clear that the IHC methods disclosed in WO2008/033495 (said IHC methods are used alternatively to the in vivo imaging method), contact the labeled antibody with the target cells only after the removal of the tissue in question (see in particular paragraph [0044], [0102] and [0103] as well as the example 1). WO2008/033495 neither discloses nor suggests to administer the antibody prior to the removal of the respective tissue sample and to detect the bound antibody by way of an IHC method.
Tissue preparation is the cornerstone of immunohistochemistry. To ensure the preservation of tissue architecture and cell morphology, prompt and adequate fixation is essential. However, inappropriate or prolonged fixation may significantly diminish the antibody binding capability as the antigenic targets are altered or even destroyed in the course of the fixation procedure. This might lead to diminished signal intensity, to a complete absence of the signal, or even worse, to alterations of the target which might result in false positive and/or false negative signals. The reliability of IHC results, therefore, depends on the possible alterations of the target during the IHC-fixation. Also other factors might influence the reliability of IHC results. It is for example standard to embed the fixed tissue in paraffin which, however, presupposes that the binding domain (e.g. an antibody) which is employed for the IHC measurement is "paraffin-permeable", i.e. it must be able to find its target in a paraffin setting. An antibody applicable for an IHC study has been optimized to fulfill such specific criteria (e.g. paraffin permeability) and therefore, may have binding characteristics different from the therapeutic antibody. Therapeutic antibodies are, generally spoken, not paraffin-reactive, and it is therefore necessary to develop antibodies, besides the therapeutically effective antibodies, which can be used for the detection of the target in IHC. This difference between the "diagnostic" and "therapeutic" antibodies, however, tends to result in problems. For example, despite the impressive clinical benefit of trastuzumab in patients with early and advanced HER2-positive breast carcinoma, not all treated patients are likely to benefit to a similar extent. Being a molecularly targeted therapy, the clinical efficacy of trastuzumab depends on the precise assessment of HER2 status in tumor specimens of candidate patients for treatment. Currently, the two most standardized approaches for HER2 status evaluation in the clinical setting are immunohistochemistry (IHC) and FISH. Despite reliable selection of patients with HER2-positive tumors using the above-mentioned methods, the response rate (RR) with single-agent trastuzumab in patients with advanced breast cancer only ranges from 19% to 34%. This is so, because the most widely used IHC methods to detect HER2 over expression can actually not document the presence of the specific target site of trastuzumab. Baker et al., 2008, Clinical Cancer Research, 14(7):2171-2179, disclose direct visualization of heterogeneous extravascular distribution of trastuzumab in human epidermal growth factor receptor type 2 overexpressing xenografts. Koyama Yoshinori et al., 2007, Neoplasia, 9(12):1021-1029, disclose *in vivo* molecular imaging to diagnose and subtype tumors through receptor-targeted optically labeled monoclonal antibodies.

There is thus a need in the art for an improved method which allows for a more accurately and/or more sensitive detection of a desired target (preferably a cellular target) and/or a subset of cells characterized by such a target.

Thus, the technical problem underlying the present invention is to provide diagnostic methods for the detection of cells ex vivo.

The present invention addresses this need and thus provides, as a solution to the technical problem, a immunohistochemistry (IHC) method for detecting a subset of cells with a binding domain which is specific for a target which characterizes said subset of cells, which method comprises detecting in a tissue section, ex vivo, said subset of cells in a tissue sample from a subject, wherein said subject is one who has received said binding domain prior to the removal of said tissue sample, wherein said binding domain is labeled and wherein said subset of cells is detected by direct immunohistochemistry.

Up to now, verification of target expression is generally not performed with the therapeutic antibody, but with an antibody appropriate for IHC. However, this approach has limitations which may lead to misinterpretations of target confirmation. Furthermore, conventional formalin fixation has disadvantages (Chu W-S et al. Modern Pathol 2005; 18: 850-863). Fixation may modify the surface antigen in such a way that binding of the IHC antibody is diminished (or enhanced) which does not reflect the clinical situation. In addition, formalin fixed paraffin embedded tissue slides have to be processed in special ways to get optimal staining with an IHC antibody (e.g. antigen retrieval by heating or incubation with special enzymes). Finally, tumor-associated surface antigens can be modulated (over-expressed, internalized or shedded) during tumor growth and metastatic spread which illustrates the necessity to evaluate the actual effectiveness of a given therapy as often as possible in order to be able to either alter the therapy (use a different binding domain) or to decide whether a given therapy is still suitable or not. Thus, a technique which allows verification of target expression and demonstration of binding of the therapeutic antibody simultaneously in vivo at the time point when therapy is initiated would be desirable and would optimize the stratification process.

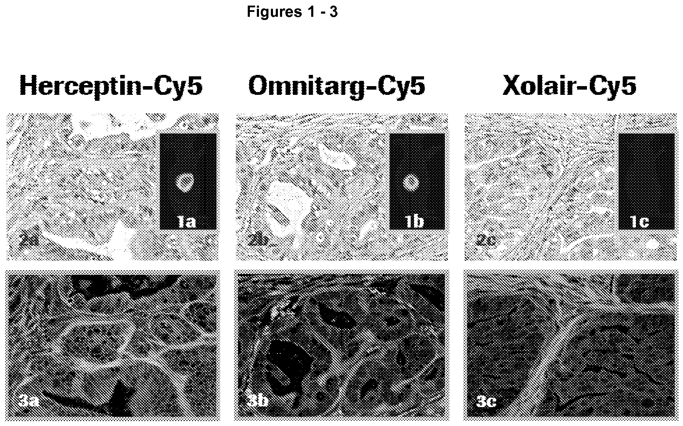

We have developed a method which addresses both issues. Therapeutic antibodies are labeled with an organic fluorophore and injected i.v. in human xenografts. In this report we demonstrate the utility of near infrared fluorescence (NIRF) to verify target expression and binding to tumor tissue. After a single i.v. injection of 50 microgram of Cy5 labeled Herceptin or Omnitarg in mice (which carry a Her2 positive tumor) a strong fluorescence signal is detectable in the tumor area after 24 to 48 hours. Subsequent analysis of explanted tumor tissue reveals that the Her2 specific antibodies bind only to tumor cells, but not to murine tissue. In contrast, no fluorescence signal is generated with a control antibody Xolair which is directed to human IgE (Fig 1-3). This approach is superior to conventional detection of target antigens by classical IHC because i) the relevant target is in its "natural environment" while it is bound by the binding domain; ii) modulation of target expression during the primary tumor growth and metastatic spread can be detected iii) explantation of tumor tissue and fixation is to be performed after the binding domain (antibody) has bound to the relevant target iv) differences in binding characteristics of therapeutic antibody and IHC antibody become obsolete.

Further embodiments of the present invention are characterized and described herein and also reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention. Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Nothing herein is to be construed as an admission that the invention is not entitled to antedate such disclosure by virtue of prior invention.

In a first aspect, the present invention relates to a immunohistochemistry (IHC) method for detecting a subset of cells with a binding domain which is specific for a target which characterizes said subset of cells, which method comprises detecting in a tissue section, ex vivo, said subset of cells with said binding domain, wherein said binding domain is to be administered to a subject which comprises or is assumed to comprise said subset of cells, prior to the removal of said subset of cells, wherein said binding domain is labeled and wherein said subset of cells is detected by direct immunohistochemistry. The methods of the invention are based on IHC-methods.
The present disclosure also relates to a method for detecting a subset of cells with a binding domain which is specific for a target which characterizes said subset of cells, which method comprises detecting, ex vivo, said subset of cells with said binding domain, wherein said subset of cells originates from a subject which subject (a) comprises or is assumed to comprise said subset of cells and (b) has been pre-treated with said binding domain.

The methods of the present invention are in vitro (or ex vivo) methods.

In a further aspect, the present disclosure relates to a method for detecting a target which characterizes a subset of cells, with a binding domain which is specific for said target, which method comprises detecting, ex vivo, said target, wherein said binding domain is to be administered to a subject which comprises or is assumed to comprise said subset of cells and/or target, prior to the removal of said subset of cells.
The present disclosure also relates to a method for detecting a target which characterizes a subset of cells, with a binding domain which is specific for said target, which method comprises detecting, ex vivo, said target, wherein said subset of cells originates from a subject which subject (a) comprises or is assumed to comprise said subset of cells and (b) has been pre-treated with said binding domain.
In a preferred embodiment of the methods of the present invention, said subset of cells is comprised within a tissue sample.

It will be understood that the binding domain is to be administered to the subject in an amount and for a period of time, which is/are sufficient to allow its binding to the target.

The present invention thus relates to IHC methods which are characterized in that the binding domain which is specific for a target, binds to the target in its natural environment, and in particular before the subset of cell characterized by that target has been removed form that subject and was subject to an IHC fixation procedure. "The natural environment" of the target is thereby for example the target in vivo (within a subject) or a cell line in its cell culture environment. The term "IHC fixation procedures" includes all the well-known steps and methods which can be carried out in order to ensure the preservation of tissue architecture and cell morphology. Such IHC fixation procedures are also exemplified herein.
An "IHC method" means the localization of targets (e.g. antigens) in tissue sections by the use of labelled binding domains as specific reagents through target - binding domain interactions that are visualized by said label. It is envisaged that said tissue sections are either thin (about 2-40 µm) slices which are taken of a tissue sample, or if the tissue sample is not very thick and is penetrable it is used whole. Said tissue sample is preferably embedded, for example in paraffin.

The present invention thus relates in a further embodiment to a method, an IHC method, comprising the step of detecting a subset of cells which is characterized by a target which is specific for that subset of cells, which target is characterized in that said target is bound by a binding domain which is specific for that target, which binding domain has bound to said target prior to the removal of that subset of cells (or tissue sample comprising said subset of cells) from a subject.

In another embodiment, the present invention relates to a method, an IHC method, comprising the step of detecting a target which is specific for a subset of cells, which target is characterized in that said target is bound by a binding domain which is specific for that target, which binding domain has bound to said target prior to the removal of that subset of cells (or tissue sample comprising said subset of cells) from a subject.

The "detection" which takes place ex vivo is carried out by standard detection techniques which are well-known to the skilled person and include, but are not limited to, any kind of suitable IHC detection techniques, such as fluorescence based microscopy, preferably near infrared based microscopy, optionally including slide scanning. Said slide scanning can be used for the subsequent 3D reconstruction.

The term "binding domain" characterizes in connection with the present invention a domain of a polypeptide which specifically binds to/interacts with a given target. Preferably, said binding domain is able to specifically bind/interact with a specific antigen or tumor target antigen or a specific group of antigens, e.g. the identical antigen or tumor target antigen in different species. Said binding/interaction is also understood to define a "specific recognition of a target". The term "binding domain" specifically includes all kinds of scaffolds (some of them are further characterized herein) which scaffolds are characterized by at least one antigen or epitope interaction site. An antigen or epitope interaction site is characterized by an antigen/epitope specific binding entity which specifically recognizes an antigen or epitope (i.e. a target of the invention). Such an antigen or epitope binding site is preferably antibody/immunoglobulin-domain based.

The term "specifically recognizing a target" or "specific for a target", means in accordance with the present invention that the binding domain, e.g. an antibody, is capable of specifically interacting with and/or binding to a target as defined herein. As used herein, the term "binds" in connection with the interaction between a target and a binding domain indicates that the binding domain associates with (e.g., interacts with or complexes with) the target to a statistically significant degree as compared to association with proteins generally (i.e., non-specific binding). Thus, the term "binding domain" is also understood to refer to a domain that has a statistically significant association or binding with a target.
The specific interaction of the antigen-interaction-site with its specific antigen may result as well in a simple binding of said site to the antigen. Moreover, the specific interaction of the binding domain/antigen-interaction-site with its specific antigen may alternatively result in the initiation of a signal, e.g. due to the induction of a change of the conformation of the antigen, an oligomerization of the antigen, etc. "Binding domains" of the present invention include but are not limited to antibodies, a domain antibody (dAb), lipocalins, affibodies, avimers, peptide aptamers, microbodies etc.

A preferred example of a binding domain in line with the present invention is an antibody or antigen binding fragment thereof, more preferably a monoclonal antibody or antigen binding fragment thereof and even more preferred a therapeutic antibody or antigen binding fragment thereof,. Said antibody or antigen binding fragment thereof is in a most preferred embodiment fluorescently labelled. Bispecific or multimeric antibodies formats are also envisaged.

The binding domain (e.g. an antibody) is to be administered to the subject before the subset of cells and/or said tissue sample comprising said subset of cells is removed and also before the detection is carried out, i.e. prior to the detection (including the fixation) and consequently also prior to the removal of said subset of cells and/or said tissue sample comprising said subset of cells. "Prior to" further means that the timeframe between the actual administration of the binding domain and the actual removal of the subset of cells and/or the tissue sample is such that, according to the circumstances, the binding domain had sufficient time to bind to its target. Said period of time depends on the target, on the binding domain, the binding characteristics of the binding domain (affinity for the target or avidity of the binding domain), the half life of the binding domain in the subject, the blood clearance of the binding domain (provided that it is administered intravenously), etc. The skilled person is however able to judge which period of time is sufficient in order to allow for a specific binding of the binding domain to the target. The same holds true for the amount of the binding domain, route of administration etc. Provided that a secondary binding domain is used in order to detect the 1^{st} binding domain (the primary binding domain) it is also envisaged that either only the 1^{st} binding domain is to be administered prior to the detection, or both, i.e. the primary and the secondary binding domain are to be administered prior to the detection (either simultaneously or sequentially), and consequently also prior to the removal of said subset of cells and/or said tissue sample comprising said subset of cells as explained above. The primary and secondary binding domains are explained in more detail herein below.

It is preferred that the primary binding domain is to be administered to the subject prior to the detection of, and consequently also prior to the removal of the subset of cells and/or said tissue sample comprising said subset of cells, and that that secondary binding domain (if employed) is used after the removal of the of said subset of cells and/or said tissue sample comprising said subset of cells.

It is more preferred that just a primary binding domain but no secondary binding domain is employed in order to detect the 1^{st} binding domain.

The subject of the present invention is a subject to whom the binding domain is to be administered prior to the removal of said subset of cells/tissue sample. Alternatively, the subject of the present invention is a subject who has received the binding domain of the invention prior to the removal of said tissues sample/subset of cells. It is envisaged that the subject of the invention already comprises the binding domains defined herein prior to the removal of the tissue sample/subset of cells. The subject of the invention comprises or is assumed to comprise said subset of cells and/or target. "Is assumed to comprise" means that it is intended to diagnose whether a subset of cells and/or target is present, absent, over-expressed or under-expressed. It is therefore not necessary that it is known beforehand (i.e. before the actual detection takes place) whether a subset of cells and/or a target of interest is present, absent, over-expressed or under-expressed. It is furthermore envisaged that the subject of the present invention is a subject comprising the binding domain of the present invention. It will be understood that the tissue sample and/or subset of cells of the invention is obtained from the subject of the invention.
The present invention thus relates to an immunohistochemistry (IHC) method for detecting a subset of cells with a binding domain which is specific for a target which characterizes said subset of cells, which method comprises detecting in a tissue section, ex vivo, said subset of cells, wherein said subset of cells (or a tissue sample as described herein) is obtained from a subject who has received the binding domain of the invention prior to the removal of said tissues sample/subset of cells, wherein said binding domain is labeled and wherein said subset of cells is detected by direct immunohistochemistry.
The route of administration depends on the circumstances and includes oral administration, but the administration is preferably parenterally, e.g., intravenously, intramuscularly, intraperitonealy, intratumorally etc.. Intravenous administration is more preferred. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

It is envisaged that the binding domain is specific for a target which characterizes a subset of cells. The term "subset of cells" denotes (a) cell(s) of interest which are characterized and/or identifiable by at least one, i.e. one, two, three, four, five, six, seven or even more target(s) which target(s) helps to distinguish this subset of cells from other cells. Said target either is or is assumed to be specific for the subset of cells.

The target may be specifically present on or in said subset of cells (expressed), it may be specifically up-regulated on/in said subset of cells (over-expressed), it may be specifically down-regulated on/in said subset of cells (under-expressed) or it may be specifically absent from the envisaged subset of cells. Thus, it will be understood that not only the presence or absence of a target may characterize the subset of cells but also alterations in the expression level of the target (e.g. over the time; responsive to a stimulus or inhibitor; in comparison to the normal/natural state of a comparable control-cell, wherein "normal/natural state of a comparable control-cell "means a control-cell which is preferably of the same kind as the cell(s) which form the subset of cells, e.g. both are epithelial cells) but which is/was derived from a different source. "A different source" includes e.g. a cell/tissue sample obtained from a healthy subject or a cell/tissue sample obtained from a distinct but obviously healthy part of the same subject wherein said different part appears to be free from associated symptoms of a disease which is characterized by said subset of cells.
It is also envisaged to define a subset of cells by a mixture of the aforementioned conditions, e.g. at least one target is specifically present while another is not etc.

"Cells of interest" means that it depends on the intention of the analysis, and therefore on the selected target(s), to determine which cells are of interest and which are not. It is for example well known that certain tumors overexpress tumor markers like β-HCG, CA 15-3, CA 19-9, CA 72-4, CFA, MUC-1, MAGE, p53, ETA, CA-125, CEA, AFP, PSA, PSMA, the ErbB family of receptors which is a subfamily of four closely related receptor tyrosine kinases: EGFR (ErbB-1), HER2/c-neu (ErbB-2), Her 3 (ErbB-3) and Her 4 (ErbB-4) etc., i.e. the "subset of cells" may than comprise cells (presumably tumor cells) which express/overexpress one or more of these tumor markers. It is likewise possible to identify a subset of cells which does not express one or all of the mentioned tumor markers - depending on the intention of the analysis.

The cells which form a subset of cells may be animal cells, cells of parasites such as worms, fungal cells, bacteria, viruses, or protozoa.

The term "animal cells" includes for example cells of a "subject" which is defined herein later. Tumor cells are preferred. Thus, in a preferred embodiment, said subset of cells consists of or comprises (a) tumor cell(s).

The term "tumor" or "tumor cells" as used herein refers to or describes the physiological condition in mammals that is typically characterized by unregulated cell growth. Examples of tumors include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, and melanoma. "Tumor cells" further includes a "solid tumor" which refers to tumors elected from the group of gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer, breast cancer being preferred.

Cell lines are also envisaged. These cell lines may be used in "isolated form". The cell lines as such are not limited, i.e. every thinkable cell which expresses a target within the meaning of the present invention is envisaged. The cell lines may be homogenous or heterogeneous. Preferably, said cell lines are derived from tumor cells.

The term "protozoa" or protozoan parasites includes for example Entamoeba histolytica or species belonging to the Apicomplexa (particularly the blood borne suborders including Adeleorina, Haemosporida and Eimeriorina, species of the genus Plasmodia being preferred), and/or endoparasites.

The term fungi includes species of Aspergillus, Candida, Cryptococcus, Histoplasma, Blastomyces, Paracoccoides, Mucor, Curvularia, Fusarium etc. including the fungal spores.

The term "bacteria" includes species which grow interlike for example species of Escherichia, Salmonella, Shigella, Klebsiella, Vibrio, Pasteurella, Borrelia, Leptospira, Campylobacter, Clostridium, Corynebacterium, Yersinia, Treponema, Rickettsia, , Chlamydia, Mycoplasma, Coxiella, Neisseria, Listeria, Haemophilus, Helicobacter, Legionella, Pseudomonas, Bordetella, Brucella, Staphylococcus, Streptococcus, Enterococcus, Bacillus, Mycobacterium, Nocardia, etc.

The term "virus" includes for example viruses of the genus Picornaviridae, Caliciviridae, Reoviridae, Togaviridae, Flaviviridae, Orthomyxoviridae, Paramyxoviridae, Rhabdoviridae, Coronaviridae, Bunyaviridae, Arenaviridae, Rteroviridae, Parvoviridae, Papovaviridae, Adenoviridae, Herpesviridae, Poxviridae, HAV, HBV, HCV, HIV, HTLV, influenza virus, herpes virus, pox virus, including all known subtypes and variations, HBV, HCV and HIV being preferred.

There is no need that the so-defined subset of cells is homogenous as such. i.e. it is thinkable that different cell types (heterogeneous cells) are grouped together, for example because all these different cells express a wanted target which characterizes or which is assumed to characterize these cells - for example cells expressing a tumor marker or cells expressing a receptor like for example cytokine or hormone receptors. It is however, also envisaged that the subset of cells is homogenous, i.e. mainly consist of one and the same cell type (e.g. epithelial cells, fibroblasts, etc.). Tumors are normally classified by the type of cell that resembles the tumor and, therefore, tumor cells are often homogenous. Examples of general categories of homogenous tumors include: carcinoma (malignant tumors derived from epithelial cells including the common forms of breast, prostate, lung and colon cancer); sarcoma (malignant tumors derived from connective tissue, or mesenchymal cells); lymphoma and leukemia (malignancies derived from hematopoietic (blood-forming) cells); germ cell tumor (tumors derived from totipotent cells - in adults most often found in the testicle and ovary; in fetuses, babies, and young children most often found on the body midline, particularly at the tip of the tailbone); blastic tumor or blastoma (a tumor (usually malignant) which resembles an immature or embryonic tissue. Many of these tumors are most common in children). All these mentioned tumors may form a subset of cells, respectively. Likewise it is possible to define a more limited subset of tumor cells within the mentioned tumors, e.g. those tumor cells which express, overexpress and/or under-express at least one target, e.g. a receptor of the ErbB family of receptors. The subset of cells is than for example a tumor cell which overexpresses a receptor of the ErbB family of receptors, e.g. the Her2/neu receptor.

Said subset of cells is either comprised in a tissue sample or it is the tissue sample as such (e.g. tumor cells derived from a tumor, cell lines which are grown in cell culture, primary cells which have been isolated and preferably also purified in order to enrich for the desired cell type). For example tumor cells and/or a (micro)metastase are frequently surrounded by healthy, i.e. non-tumorigenic tissue, i.e. the tumor cells could then form a subset of cells within the healthy tissue. A tissue sample thereby could comprise a subset of healthy cells and a subset of tumorigenic cells. Within said subset of tumor cells, it might be possible to define a further subset of tumor cells, e.g. such tumor cells which express/overexpress HER2/neu. It is also envisaged that for example viruses or other pathogenic species (bacteria, protozoa etc.) are comprised by a tissue sample. Such bacteria could than form a subset of cells within this tissue sample or the cells which comprise, for example intracellular bacteria, could form a subset of cells within the tissues sample. As mentioned before, it is also envisaged that the tissue sample is entirely composed of a subset of cells. It will be understood that the terms "tissue", "tissue sample", "tissue section" etc. are used interchangeably. Moreover, it is also envisaged that the methods of the present invention can carried out with subset of cells (which may be comprised in a tissue sample).

The tissue sample may be obtained via biopsy such as needle biopsy, surgical biopsy, bone marrow biopsy etc.

The term "target" or "target which characterizes said subset of cells" includes all structures/antigens/epitopes to which a binding domain binds and which are specific, or which are assumed to be specific, for a desired subset of cells. "Specific" means that this target characterizes said subset of cells, i.e. it is mainly present on (or in) these cells or mainly absent or mainly over- or under-expressed on or in these cells. "Mainly" shall thereby reflect the fact that in biological systems it is nearly impossible to achieve a situation where a desired subset of cells remains unchanged over the time. The target may be associated with a biological state, such as a disease or disorder as well as the presence of a pathogen. When a target is "associated with" a certain biological state, the presence or absence of the target or the presence or absence of a certain amount of target can identify the biological state.

The "target" in the context of the present invention specifically includes a protein, a peptide, an enzyme, a nucleic acid, a polysaccharide, a lipid, a receptor, a cellular target, and/or a tumor antigen or any other kind of structure/antigen/epitope whose presence (qualitatively) and/or whose amount (quantitatively) is of interest and characterizes a subset of cells. Preferably, said target (its presence, absence or amount) has a prognostic value for a specific disease, preferably for cancer.

A "cellular target" includes all kinds of epitope/antigen/structure, preferably on the cell surface, which characterize a subset of cells, such as CAMs like NCAM, ICAM-1, VCAM-1, PECAM-1, L1, CHL1, MAG, integrins such as αvβ3 and αvβ5 integrins, selectins, CD antigens such as CD1d, which may be used for the targeting of, e.g., dendritic cells, intestinal epithelial cells, B cell subset, NK T cell subset; CD 11a,b,c,d; CD14 and CD16/18, which may be used for the targeting of, e.g., macrophages; CD23, which may be used for the targeting of e.g., activated mature B cells expressing IgM or IgD (particularly mantle cells), activated monocytes/macrophages, T cell subsets, platelets, eosinophils, Langerhans cells, follicular dendritic cells, or intestinal epithelium; CD54 (also known as ICAM-1), which may be used for the targeting of, e.g., B and T cells and B cell precursors, monocytes, osteoclasts, endothelial cells, and various epithelial cells; CD57, which may be used for the targeting of, e.g., cells of the NK subset, T cell subset, neuroectodermal tissue, retina, brain, prostate, renal proximal tubules; CD64 (also called Fc gamma RI), which may be used for the targeting of antigen presenting cells including macrophages/monocytes, activated granulocytes, dendritic cells or early myeloid cells; CD91 (also known as Low density lipoprotein receptor-related protein 1 (LRP1); also called alpha-2-macroglobulin receptor), which may be used for the targeting of fibroblasts, dendritic cells, macrophages, liver, brain or lung tissue as well as CD-20, CD-45. Furthermore, the term relates to anti-CD antibodies, to antigens expressed by cancer stem cells of different origin (breast, colon, lung, prostate, ovarian, liver and pancreatic), to molecular danger signals, TLRs, bacterial toxins, e.g. 'trapo' for nerve cells as described in WO 2006/114308or DEC-205, which is typically present on dendritic cells. In addition, the term relates to a vascular-homing peptide, which may be specific for certain organs or tissues, like e.g. brain, kidney, lung, skin, or heart. More preferably, the term relates to such peptides as mentioned in Arap, W. et al. Proc. Natl Acad Sci. U.S.A., 99:1527-1531 (2002); Rajotte, D. et al., J. Clin Invest., 102:430-437 (1998); Pasqualini, R., and Ruoslahti, E. (2002) Nat. Rev. Cancer 2:83; Rajotte, D. and Ruoshlati, E., J. Biol. Chem. 274:11593-11598 (1999); Essler, M., and Ruoshlati, E., Proc. Natl Acad. Sci. U.S.A., 99:2252-2257 (2002).

The term "receptor-molecules" or "receptor" relates to protein on the cell membrane or within the cytoplasm or cell nucleus that binds to a ligand and typically transduces a signal, such as metabotropic receptors, G protein-coupled receptors, muscarinic acetylcholine receptors, adenosine receptors, adrenoceptors, GABA receptors, angiotensin receptors, cannabinoid receptors, cholecystokinin receptors, dopamine receptors, glucagon receptors, metabotropic glutamate receptors, histamine receptors, olfactory receptors, opioid receptors, chemokine receptors, calcium-sensing receptor, somatostatin receptors, serotonin receptors, secretin receptors, Fc receptors or receptor tyrosine kinases (RTK). The receptor tyrosine kinase family consists of high affinity cell surface receptors for many polypeptide growth factors, cytokines and hormones, and comprises *inter alia* the ephrine receptors (Eph receptor family), the RET receptor family; the VEGF-receptor family comprising *inter alia* VEGFR1 (Flt-1), VEGFR2 (Flk-1/KDR) and VEGFR4 (Flt-4), and the fibroblast growth factor receptor family exemplified by FGFR1, FGFR2, FGFR3, FGFR4 and FGFR6. The ErbB family of receptors which is a subfamily of four closely related receptor tyrosine kinases: EGFR (ErbB-1), HER2/c-neu (ErbB-2), Her 3 (ErbB-3) and Her 4 (ErbB-4) is also included and specifically preferred. The term "nucleic acid" refers to any nucleic acid known to the person skilled in the art, e.g. a polynucleotide like DNA, RNA, single stranded DNA, cDNA, PNA or derivatives thereof.

The term "protein" includes any polypeptide of interest, including therapeutically active proteins, enzymes, marker proteins etc.

The term "peptide" denotes a molecule formed by linking at least two amino acids (preferably alpha-amino acids), i.e. two, three, four, five, six, seven, eight, nine, ten, eleven, twelve, thirteen, fourteen, fifteen and even more, up to 30, amino acids, by amide bonds

The term "enzymes" is meant to include catalytically active proteins which are, or which are assumed to be, specific for a subset of cells, like for example metalloproteases which are frequently over-expressed in tumor cells etc.

The term "lipid" is meant to include all kinds of lipids which are able to characterize a subset of cells, and includes lipid messengers that binds a protein, such as a receptor, kinase or phosphatase, which in turn mediate the effects of these lipids on specific cellular responses.

The term "polysaccharide" means polymeric carbohydrate structures, formed of repeating units (either mono- or di-saccharides) joined together by glycosidic bonds and includes polysaccharides which are presented on the surface of cells (e.g. MUC-1), polysaccharides which form or are part of bacterial or viral capsules or envelopes, lipopolysaccharide (LPS) of gram negative bacteria, etc.

The term "tumor-antigen" which is exchangeable with "tumor-marker" includes tumor specific antigens and tumor associated antigens, preferably tumor associated antigens which are responsible for primary tumor growth and metastasis. "Tumor antigens" are exemplified by (but not limited to) β-HCG, CA 15-3, CA 19-9, CA 72-4, CFA, MUC-1, MAGE, ras, p53, ETA, CA-125, CEA, AFP, PSA, PSMA, Ep-CAM, CD33, CD44v6, MCSP, CD30, the ErbB family of receptors which is a subfamily of four closely related receptor tyrosine kinases: EGFR (ErbB-1), HER2/c-neu (ErbB-2), Her 3 (ErbB-3) and Her 4 (ErbB-4), angiopoietins and their receptors, VEGFs and their receptors, CD9; CDCP1; CSF1R; CYR61; HER3; HPN (= Hepsin); TDGF1; TROP2; CD44; IGF1R; TWEAK; EGFR and PLGF and/or c-met, to name some.

Also bacteria, viruses or other pathogenic organisms mentioned herein can form a target, preferably when these pathogenic organisms are comprised by a cell (host cell). The host cells are than characterized by said target (bacterium, virus).

Targets which are presented on the surface of the subset of cells are particularly preferred (either as a single target or combinations thereof).

Targets which are of diagnostic value are even more preferred. "Diagnostic value" means that it is already known or will be known in the future that the presence or absence or up-regulation or down-regulation of a target has prognostic value for a certain disease (like HER2/neu for breast cancer).

The term "ex vivo", which is interchangeable with "in vitro" refers to activities conducted in cells in a controlled environment. As used herein and in the art, this term is often used interchangeably with "in culture", which may refer to cells in a cell culture or cells in an organ culture.
Thus, in another aspect, the present invention relates to an IHC method for detecting a subset of cells and/or for detecting a target which characterizes said subset of cells, with a binding domain which is specific for a target which characterizes said subset of cells, which method comprises detecting in a tissue section, in vitro, said subset of cells, wherein said binding domain has bound its target prior to the detection of said subset of cells and/or prior to the detection of said target, wherein said binding domain is labeled and wherein said subset of cells is detected by direct immunohistochemistry.

Said subset of cells is comprised within a tissue sample.

The binding domain of the present invention is labeled.

A label refers in the context of the invention to a compound or composition detectable by spectroscopic, photochemical, biochemical, immunochemical, or chemical means.

The label may be directly or, as disclosed herein, indirectly detectable.

"Directly" means that the label as such generates the signal such as a radioactive, chromogenic, or fluorescent signal. Direct labels include radiolabels; fluorescent label, electron-dense reagents; etc. The direct label has or generates a measurable signal that can be used to quantify and/or detect (qualitatively) the bound binding domain. A fluorescent label is the preferred direct label.
In some embodiments of the present invention, said detection is by direct immunohistochemistry and comprises detecting the presence of said (preferably fluorescently) labeled binding domain. Said binding domain is more preferably an antibody.

It will be understood that the primary binding domain is directly labelled if no secondary binding domain is employed.

"Indirectly" means that the label is, for example, bound by another entity which as such is then detectable (detection entity). Indirect detection or indirect label involves the binding of a second directly or indirectly detectable binding domain to the indirect label. For example, the indirect label of the binding domain of the disclosure can be the ligand of a binding partner, such as biotin, which is a binding partner for streptavidin, or a nucleotide sequence, which is the binding partner for a complementary sequence, to which it can specifically hybridize etc. "Indirect labelling" is therefore characterized in that the primary binding domain is manipulated such that it can be detected by a second binding domain (sometimes also denoted detection entity) which is specific for that manipulation (e.g. a biotin label).

In an illustrative example said binding domain is an antibody (primary antibody) and said detection entity is a secondary antibody which specifically reacts with the (label of the) primary antibody.

It is also envisaged that indirect and direct labelling is mixed. For example, already the direct label is able to generate a signal (e.g. a fluorescent label like FITC) but the secondary binding domain, which is also labelled, e.g. with a direct label binds specifically to that label (anti FITC antibody) binds to the label of the primary signal and, thereby, may increase the detectable signal. Such means and methods are well-known to the skilled person, in particular to practitioners in the field of immunochemistry.

It is preferred that at least 10%, of the population of binding domains which are/are to be administered to the subject (or which are comprised by the subject), are labeled either with a direct or with an indirect label. Preferably more then 50, 60, 70, 80, 90% or even 100% of the population of binding domains which are/are to be administered to the subject, are labeled with either a direct or with an indirect label.

In another illustrative example of the disclosure, the primary binding domain (e.g. an antibody) is not labeled as such but is detected/detectable by way of a secondary binding domain (detection entity) which binds to the primary binding domain (for example a primary non-labeled antibody raised in mouse is detected by a second, labeled antibody, which was raised in another species and specifically binds to mouse antibodies (e.g. goat anti-mouse)). The secondary binding domain is then directly or indirectly labeled. Such antibody detection sandwiches are well-established and the skilled person will have no problem to generate/establish or create such systems.

The most common fixative used for immunohistochemistry is paraformaldehyde, which is frequently used in diverse buffers containing about 1 to 5% paraformaldehyde. Specific buffers which are based on paraformaldehyde are exemplified in the following:
a) 4% paraformaldehyde in 0.1M phosphate buffer
b) 2% paraformaldehyde with 0.2% picric acid in 0.1M phosphate buffer
c) PLP (paraformaldehyde, lysine, paraformaldehyde) fixative: e.g. 4% paraformaldehyde, 0.2% periodate and 1.2% lysine in 0.1M phosphate buffer
d) 4% paraformaldehyde with 0.05% glutaraldehyde.

These buffers are not intended to limit the invention but simply illustrate specific conditions which are normally applied to achieve a sufficient fixation of tissue. The standard fixation time is about 5, 10, 15, 20, 30 min to overnight. The so-treated tissue is frequently subject to a subsequent paraffin embedding protocol, followed by the incubation in organic solvents like for example xylene and ethanol treatment. The sample is then normally hydrated by placing it in 95%, 70%, 50%, 30% alcohol (e.g.

ethanol) for several minutes each. There is, however, no standard protocol for IHC, i.e. the protocol will vary depending on the target, the tissue, the antibodies used etc. All this is known to the skilled person and routinely handled without further ado. Specific protocols are disclosed for example in the internet (searchable with the string IHC protocols in a search machine like Google etc.). Some antigens will not even survive moderate amounts of aldehyde fixation. Under this condition, tissues are often fresh frozen in liquid nitrogen and cut with a cryostat. The sections are kept frozen at -20 C or lower until fixation with cold acetone or alcohol.

It is thus envisaged that the label which is employed in the context of the present invention withstands the IHC procedure, e.g. the fixation, the paraffin embedding the hybridization etc. A label "withstands the IHC procedure" if it is still detectable after it has been subject to a IHC protocol. For example: some labels might be inactivated by heat (paraffin embedding) or by organic solvents, or by the fixation buffers etc. and are no longer able to generate a detectable signal, either because the signal is to low or because the signal is no longer present. The resistance of a label will thus depend on the specific IHC protocol which is used. Thus, it might be that one label is inactivated in the course of one specific IHC protocol but still useable upon employing another IHC protocol.
Preferably, the label of the invention withstands the fixation, more preferably it withstands aldehyde fixation with formalin. "Withstands" means in this regard that it is still detectable after said procedure, i.e. that it does preferably not loose more then 50% of its detection capability.

It is envisaged that also the primary binding domain and, if employed prior to the detection also the secondary binding domain, withstand the IHC procedure.

A "fluorescent label" as used herein characterizes a molecule which comprises a fluorophore. A fluorophore, which is sometimes also termed fluorochrome, is a functional group in a molecule which will absorb energy of a specific wavelength and re-emit energy at a different wavelength. Said different wavelength, when compared to the said specific (predetermined) wavelength, is re-emitted with a wavelength which is distinguishable from the specific (predetermined) wavelength, for example it is re-emitted with a longer wavelength or with a shorter wave-length, however in the latter case with decreased intensity. The amount and wavelength of the emitted energy depends on both the fluorophore and the chemical environment of the fluorophore.
Methods for coupling fluorescent labels including NIR fluorescence labels are well known in the art. The conjugation techniques of these labels to an antibody have significantly matured during the past years and an excellent overview is given in Aslam, M., and Dent, A., Bioconjugation (1998) 216-363, London, and in the chapter "Macromolecule conjugation" in Tijssen, P., "Practice and theory of enzyme immunoassays" (1990), Elsevier, Amsterdam.
Appropriate coupling chemistries are known from the above cited literature (Aslam, supra). The fluorescent label, depending on which coupling moiety is present, can be reacted directly with the antibody either in an aqueous or an organic medium. The coupling moiety is a reactive group or activated group which is used for chemically coupling of the fluorochrome label to the antibody. The fluorochrome label can be either directly attached to the antibody or connected to the antibody via a spacer to form a NIR fluorescence label conjugate comprising the antibody and a NIR fluorescence label. The spacer used may be chosen or designed so as to have a suitably long *in vivo* persistence (half-life) inherently.

The fluorescent label is preferably selected from the group consisting of quantum dot agents, fluorescent dyes, pH-sensitive fluorescent dyes, voltage sensitive fluorescent dyes, and fluorescent labeled microspheres, fluorescent dyes being preferred.

"Quantum dot agents" or "Quantum dots", also known as nanocrystals, are a special class of materials known as semiconductors, which are crystals composed of periodic groups of II-VI, III-V, or IV-VI materials.

"Fluorescent protein" includes for example green fluorescent protein (GFP), CFP, YFP, BFP either enhanced or not. Further fluorescent proteins are described in Zhang, Nat Rev Mol Cell Biol. 2002, 12, pages 906-18 or in Giepmans, Science. 2006, 312, pages 217-24.

"Fluorescent dyes" includes all kinds of fluorescent labels including but not limited to, Fluorescein including all its derivatives like for example FITC; Rhodamine including all its derivatives such as tetramethylrhodamine (TAMRA) and its isothiocyanate derivative (TRITC), sulforhodamine 101 (and its sulfonyl chloride form Texas Red), Rhodamine Red, and other derivatives of rhodamine which include newer fluorophores such as Alexa 546, Alexa 555, Alexa 633, DyLight 549 and DyLight 633); Alexa Fluors (the Alexa Fluor family of fluorescent dyes is produced by Molecular Probes); DyLight Fluor which is a family of fluorescent dyes are produced by Dyomics, ATTO Dyes, which represent a series of fluorescent labels and dyes manufactured by ATTO-TEC GmbH in Siegen, WO/2007/067978 Japan); LaJolla Blue (Diatron, Miami, Fla.); indocyanine green (ICG) and its analogs (Licha et al., 1996, SPIE 2927:192-198; Ito et al., U.S. Pat. No. 5,968,479); indotricarbocyanine (ITC; WO 98/47538), and chelated lanthanide compounds. Fluorescent lanthanide metals include europium and terbium. Near-infrared (NIR) fluorescence label are also envisaged. NIR fluorescence labels with excitation and emission wavelengths in the near infrared spectrum are used, i.e., 640-1300 nm preferably 640-1200 nm, and more preferably 640-900 nm. Use of this portion of the electromagnetic spectrum maximizes tissue penetration and minimizes absorption by physiologically abundant absorbers such as hemoglobin (<650 nm) and water (>1200 nm). Ideal near infrared fluorochromes for *in vivo* use exhibit:
(1) narrow spectral characteristics,
(2) high sensitivity (quantum yield),
(3) biocompatibility,
(4) decoupled absorption and excitation spectra, and
(5) photo stability.

Various near infrared (NIR) fluorescence labels are commercially available and can be used to prepare a fluorescent entity according to this invention. Exemplary NIRF labels include the following: Cy5.5, Cy5 and Cy7 (Amersham, Arlington Hts., IL; IRD41 and IRD700 (LI-COR, Lincoln, NE); NIR-I, (Dejindo, Kumamoto, Japan); LaJolla Blue (Diatron, Miami, FL); indocyanine green (ICG) and its analogs (Licha, K., et al., SPIE-The International Society for Optical Engineering 1996; Vol. 2927: 192-198; US 5,968,479); indotricarbocyanine (ITC; WO 98/47538); and chelated lanthanide compounds and SF64, 5-29, 5-36 and 5-41 (from WO 2006/072580). Fluorescent lanthanide metals include europium and terbium. Fluorescence properties of lanthanides are described in Lackowicz, J. R., Principles of Fluorescence Spectroscopy, 2nd Ed., Kluwer Academic, New York, (1999).

"Fluorescent microspheres" are described in great detail in WO/2007/067978.
It is known that the amount and wavelength of the energy emitted by a fluorescent dye depends on both the fluorophore and the chemical environment of the fluorophore. It follows that fluorescent dyes may react pH-sensitive or voltage sensitive, i.e. they are activatable by such changes in the chemical environment. Further activatable fluorescent labels are described for example in great detail in US 2006/0147378 A1, US 6592847, US 6,083,486, WO/2002/056670 or US 2003/0044353 A1.

Fluorescent dyes are preferred in the context of the present invention. Particularly preferred are fluorescent dyes of the Cy, Alexa and Dylight family.

The subject is within the context of the present invention an animal. Preferably, said subject is a vertebrate, more preferably a mammalian. It is more preferred that said mammalian subject is a human, a monkey such as cynomolgus, a mouse, a rat, a guinea pig, a rabbit, a horse, a camel, a dog, a cat, a pig, a cow, a goat or a fowl. A human subject is most preferred. A non-human subject may represent a model of a particular disease or disorder. It is also envisaged that the subject of the present invention comprises a xenograft, preferably human tumor cells.

The binding domain of the present invention preferably is or comprises an epitope binding domain.

Preferably, said epitope binding domain is an antibody or an antigen binding fragment thereof.

The term "antibody" refers to a monoclonal or a polyclonal antibody (see Harlow and Lane, "Antibodies, A Laboratory Manual", CSH Press, Cold Spring Harbor, USA, 1988) which binds to a target, or a derivative of said antibody which retains or essentially retains its binding specificity. Preferred derivatives of such antibodies are chimeric antibodies comprising, for example, a mouse or rat variable region and a human constant region. The term "antibody" also comprises bifunctional (bispecific) antibodies and antibody constructs, like single-chain Fvs (scFv) or antibody-fusion proteins. The term "scFv fragment" (single-chain Fv fragment) is well understood in the art and preferred due to its small size and the possibility to produce such fragments recombinantly. Said antibody or antibody binding portion is a human antibody or a humanized antibody. The term "humanized antibody" means, in accordance with the present invention, an antibody of non-human origin, where at least one complementarity determining region (CDR) in the variable regions such as the CDR3 and preferably all 6 CDRs have been replaced by CDRs of an antibody of human origin having a desired specificity. Optionally, the non-human constant region(s) of the antibody has/have been replaced by (a) constant region(s) of a human antibody. Methods for the production of humanized antibodies are described in, e.g., EP-A1 0 239 400 and WO90/07861. The term antibody or antigen binding fragment thereof also includes heavy chain antibodies and the variable domains thereof, which are mentioned in WO 94/04678, WO 96/34103 and WO 97/49805, WO 04/062551, WO 04/041863, WO 04/041865, WO 04/041862 and WO 04/041867; as well as domain antibodies or "dAb's", which are based on or derived from the heavy chain variable domain (VH) or the light chain variable domain (VL) of traditional 4 chain antibody molecules (see, e.g., Ward et al. 1989 Nature 341, 544-546).

The term "antigen binding fragment" as used herein refers to fragments of the antibodies as specified herein which retain or essentially retain the binding specificity of the antibodies like, separated light and heavy chains, Fab, Fab/c, Fv, Fab', F(ab')2. An antigen-binding fragment may comprise a light chain variable region (VL) and a heavy chain variable region (VR) of an antibody; however, it does not have to comprise both. Fd fragments, for example, have two VH regions and often retain antigen-binding function of the intact antigen-binding fragment.

The term "epitope binding domain" includes, besides antibodies or antigen binding fragments thereof, other binding entities which bind to (specifically bind to) a target. The term "epitope binding domain" includes, for example, a domain that (specifically) binds an antigen or epitope independently of a different V region or domain, this may be a domain antibody (dAb), for example a human, camelid or shark immunoglobulin single variable domain or it may be a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; transferrin (trans- body); ankyrin repeat protein (DARPin); peptide aptamer; C-type lectin domain (Tetranectin); human γ-crystallin and human ubiquitin (affilins); PDZ domains; scorpion toxin kunitz type domains of human protease inhibitors; and fibronectin (adnectin); which has been subjected to protein engineering in order to obtain binding to a ligand other than the natural ligand. CTLA-4 (Cytotoxic T Lymphocyte-associated Antigen 4) is a CD28-family receptor expressed on mainly CD4+ T-cells. Its extracellular domain has a variable domain- like Ig fold. Loops corresponding to CDRs of antibodies can be substituted with heterologous sequence to confer different binding properties. CTLA-4 molecules engineered to have different binding specificities are also known as Evibodies. For further details see Journal of Immunological Methods 248 (1-2), 31-45 (2001).
Lipocalins are a family of extracellular proteins which transport small hydrophobic molecules such as steroids, bilins, retinoids and lipids. They have a rigid β-sheet secondary structure with a numer of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633.
An affibody is a scaffold derived from Protein A of Staphylococcus aureus which can be engineered to bind to antigen. The domain consists of a three-helical bundle of approximately 58 amino acids. Libraries have been generated by randomisation of surface residues. For further details see Protein Eng. Des. Sel. 17, 455-462 (2004) and EP1641818A1.
Avimers are multidomain proteins derived from the A-domain scaffold family. The native domains of approximately 35 amino acids adopt a defined disulphide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007).
A transferrin is a monomeric serum transport glycoprotein. Transferrins can be engineered to bind different target antigens by insertion of peptide sequences in a permissive surface loop. Examples of engineered transferrin scaffolds include the Transbody. For further details see J. Biol. Chem 274, 24066-24073 (1999).
Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two α-helices and a β-turn. They can be engineered to bind different target antigens by randomising residues in the first α-helix and a β-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.
Fibronectin is a scaffold which can be engineered to bind to antigen. Adnectins consists of a backbone of the natural amino acid sequence of the 10th domain of the 15 repeating units of human fibronectin type III (FN3). Three loops at one end of the β-sandwich can be engineered to enable an Adnectin to specifically recognize a therapeutic target of interest. For further details see Protein Eng. Des. Sel. 18, 435- 444 (2005), US20080139791, WO2005056764 and US6818418B1.
Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).
Microbodies are derived from naturally occurring microproteins of 25-50 amino acids in length which contain 3-4 cysteine bridges - examples of microproteins include KalataBI and conotoxin and knottins. The microproteins have a loop which can be engineered to include up to 25 amino acids without affecting the overall fold of the microprotein. For further details of engineered knottin domains, see WO2008098796.
Other epitope binding domains include proteins which have been used as a scaffold to engineer different target antigen binding properties include human γ-crystallin and human ubiquitin (affilins), kunitz type domains of human protease inhibitors, PDZ-domains of the Ras-binding protein AF-6, scorpion toxins (charybdotoxin), C-type lectin domain (tetranectins) are reviewed in Chapter 7 - Non-Antibody Scaffolds from Handbook of Therapeutic Antibodies (2007, edited by Stefan Dubel) and Protein Science 15:14-27 (2006). Epitope binding domains of the present invention could be derived from any of these alternative protein domains. Examples of further "epitope binding domains" are receptors (specifically binding to their ligand), lectins (specifically binding to polysaccharides), zinc fingers and leucine zippers (binding to nucleic acids), enzymes (specifically binding to their substrate), viruses and bacteria (for example specifically binding to their target cells), nucleic acids (specifically hybridizing to each other) etc.

It is envisaged that in some embodiments of the present invention said binding domain is therapeutically active.

"Therapeutically active" means that the binding domain as such is able to or is though to be able to exert a therapeutically effect in the subject, like, for example, a therapeutically effective antibody which alleviates the symptoms or the cause of a disease, which treats a disease, is able to prevent the onset of a disease etc.

In some embodiments, said therapeutically active binding domain is selected from the group consisting of alemtuzumab, apolizumab, cetuximab, epratuzumab, galiximab, gemtuzumab, ipilimumab, labetuzumab, panitumumab, rituximab, trastuzumab, nimotuzumab, mapatumumab, matuzumab, rhMab ICR62, rhMab B-Ly1 and pertuzumab.

In some embodiments it is also envisaged that said subject has received said binding domain in a therapeutically effective dose prior to the removal of said tissue sample and/or subset of cells, i.e. said subject of the invention already comprises a therapeutically effective amount of said binding domain prior to the remove of the tissue sample/subset of cells. The subject of the present invention is alternatively a subject who has received the binding domain of the invention in a therapeutically effective dose prior to the removal of said tissues sample/subset of cells.

By way of the present invention it is now possible to combine two different capabilities of a binding domain, namely (a) its capability to detectably label a target via IHC detection technique and (b) its capability to act in a therapeutically active manner, at the same time. Thus, the detectably labeled marker which can be used in IHC and the therapeutically active agent could be one and the same entity. In particular, it is now possible to employ each and every therapeutic antibody in an IHC setting, thereby increasing the reliability of the IHC results. It is for example standard to "grade" certain tumors, e.g. depending on the expression level of a tumor marker such as HER2/neu - the prognostic relevance of such a measurement can be improved by way of the embodiments of the present invention because the measurement is now carried out with the therapeutic antibody which will be applied subsequently (or which was already applied beforehand). The resulting IHC measurement thereby reflects more accurately whether a certain tumor target (which is also "used" by the therapeutic antibody) is still there or not and consequently, whether one may assume that a subsequent therapy with the very same antibody will in all likelihood be successful or not (e.g.: if the target which is used by that therapeutic antibody is still there then one may speculate that the therapy with the very same antibody should have a better expectation of success). It is thus a significant advantage to use the same antibody for two different purposes. There is also no need to develop specific antibodies which work in an IHC setting because the antibody has already bound to its target before the subset of cells/tissue sample is subject of a fixation etc. The binding reaction antibody-target, therefore, more closely reflects the actual situation.

In addition, formalin fixed paraffin embedded tissue slides have to be processed in special ways to get optimal staining with an IHC antibody (it is for example necessary to retrieve/unmask the antigen I by heating and/or incubation with special enzymes). Such a specialized treatment is not necessary upon applying the teaching of the present invention as the antigenic epitopes are presented and bound within the subject or within a cell culture setting, thereby preventing or diminishing the risk that the antigen is masked (and therefore either not or erroneously detected by the binding domain) during the fixation procedure, thereby potentially destroying or altering epitope which indicate the presence, absence or degree of expression of that target.

The present invention also relates to the use of a binding domain as defined herein in a method defined herein.

The present invention also relates to the use of a binding domain as defined herein for the preparation of a composition to be used in a method defined herein. Said composition is preferably diagnostic composition, however pharmaceutical compositions are also envisaged.

The present invention also relates to a binding domain as defined herein to be used in a method defined herein. Thus, in a further aspect, the present invention relates to a binding domain which is specific for a target which target characterizes a subset of cells, for use in an immunohistochemistry (IHC) method of the invention, which IHC method comprises detecting in a tissue section, ex vivo, said subset of cells, wherein said binding domain is to be administered to a subject which comprises or is assumed to comprise said subset of cells, prior to the removal of said subset of cells wherein said binding domain is labeled and wherein said subset of cells is detected by direct immunohistochemistry. The present invention also relates to a binding domain which is specific for a target which target characterizes a subset of cells, for use in an immunohistochemistry (IHC) method for detecting a subset of cells, which method comprises detecting in a tissue section, ex vivo, said subset of cells, wherein said subset of cells (or a tissue sample as described herein) is obtained from a subject who has received the binding domain of the invention prior to the removal of said tissues sample/subset of cells, wherein said binding domain is labeled and wherein said subset of cells is detected by direct immunohistochemistry.

In some embodiments of the present invention, said subset of cells is detected by direct immunohistochemistry.

Immunohistochemistry (IHC) is the localization of targets (e.g. antigens) and/or subsets of cells presenting a target in tissue sections by the use of binding domains which are either directly labeled (direct IHC) or indirectly labeled (indirect IHC), which binding domains react with their target through specific target -- binding-domain interactions. These interactions are then visualized by the mentioned label.
There are mainly two strategies used for the immunohistochemical (IHC) detection of antigens in tissue, the direct method and the indirect method. The direct method of immunohistochemical staining uses one directly labelled binding domain, which binds directly to the target being stained for. The direct method is thus a one-step staining method, and involves e.g. a labelled antibody (e.g. FITC conjugated antiserum) reacting directly with the antigen in tissue sections. This technique utilizes only one antibody and the procedure is therefore simple and rapid. It is known that this direct IHC method, when carried out in the "conventional format" as it was used prior to the present invention has problems with sensitivity due to little signal amplification. This is presumably due to the fact that the target as such is already altered in the course of the fixation step which dramatically decreases the amount of "detectable" target and, consequently also decreases the amount of binding domain which might bind to and thereby detect the target. However, much to our surprise, it could be demonstrated that by applying the teaching of the present invention, i.e. by reacting the directly labelled binding domain with the target prior to the detection, and thereby prior to the removal of the tissue and also prior to the fixation of the tissue, it is indeed possible to detect the target even with a directly labelled primary binding domain (i.e. without the need to "amplify" the signal by way of a second binding domain specifically binding to the primary binding domain).

In other illustrative examples of the present disclosure said subset of cells is detected by indirect immunohistochemistry.The indirect method of immunohistochemical staining uses one binding domain against the target being probed for, and a second, labelled, binding domain against the first. Methods are envisaged, wherein said binding domain is an antibody (primary antibody) and said detection entity is a secondary antibody which specifically reacts with the (label of the) primary antibody.
The indirect method involves a primary antibody (first layer - normally unlabeled) which reacts with target, and a labeled secondary binding domain (second layer) which reacts with the primary binding domain. The secondary binding domain is for example raised against the IgG of the animal species in which the primary antibody has been raised.

It is envisaged that in some embodiments of the methods of the present invention, said immunohistochemistry is characterized by the following steps:
(a) providing a means (for example a slide) comprising said tissue sample comprising the subset of cells and/or said subset of cells to which the binding domain has bound to;
(b) optionally fixing said tissue sample;
(c) optionally dehydrating said tissue sample;
(d) optionally allowing the tissue sample to be paraffinized;
(e) directly detecting the binding domain and thereby the subset of cells.

"Fixing" or "fixation" means a fixation procedure which is suitable to prepare the target/subset of cells/tissue sample comprising said subset of cells for a subsequent IHC procedure. A "fixation" is particularly carried out in order to ensure the preservation of tissue architecture and cell morphology. Suitable fixation conditions are well-known and also disclosed herein. Alternatively, it is also envisaged that the tissue/subset is preserved by way of deep-freezing (e.g. in liquid nitrogen).
All the above pre-treatment steps/measures are within the scope of the term "fixation", i.e. fixation specifically includes fixation with fixing agents like formaldehyde, paraformaldehyde; and/or deep-freezing of the tissue sample/subset of cells, and/or optionally also the embedding of the tissue/subset of cells in paraffin or similar agents. It must be understood that the gist of the present invention lies in the surprising finding that it is advantageous that the binding domain (e.g. the primary antibody which is specific for a target) is allowed to bind to its target before the tissue/subset etc. presenting said target is subject to a fixation procedure, as the fixation procedure might effect the amount and/or quality of the target thereby altering the result in an unwanted fashion.

The tissue/subset can also be paraffinized (usually after the fixation).

Means and methods to put the different IHC protocols into practice are well-known to the skilled person and have been, and will/can be adapted to the specific tissue/subset of cells/target which is of interest, without further ado. Three exemplary protocols are listed below, which protocols are however merely illustrative and are not intended to limit the scope of the present invention.

### A. Cell Lines

Grow cultured cells on sterile glass cover slips or slides overnight
Wash briefly with PBS
Fix as desired. Possible procedures include:
10 minutes with 10% formalin in PBS (keep wet)
5 minutes with ice cold methanol, allow to air dry
5 minutes with ice cold acetone, allow to air dry
Wash in PBS

### B. Frozen Sections

Snap frozen fresh tissues in liquid nitrogen or isopentane pre-cooled in liquid nitrogen, embedded in O.C.T. compound. Store frozen blocks at - 80 °C.
Cut about 4-8 um thick cryostat sections and mount on superfrost plus slides or gelatin coated slides. Store slides at - 80 °C until needed.
Before staining, warm slides at room temperature for 30 minutes and fix in ice cold acetone for 5 minutes. Air dry for 30 minutes.
Wash in PBS

### C. Paraffin Sections

Deparaffinize sections in xylene, 2x5min.
Hydrate with 100% ethanol, 2x3min.
Hydrate with 95% ethanol, 1min.

It is preferred that the fixation procedure is carried out as follows:
Deparaffinize sections in xylene, about 3 x about 1 min;
Hydrate with 100% ethanol, about 2x about 1 min;
Hydrate with 90% ethanol, about 1min;
Hydrate with 80% ethanol, about 1min;
Hydrate with 70% ethanol, about 1min;
Water about 1 min.

Staining with Mayer's Hematoxylin for about 1 min
Wash with flowing water for about 0,5 min to 2 min
Water about 10 seconds
0,1% Eosin for about 1 minute

Hydrate with 70% ethanol, about 5 seconds;
Hydrate with 80% ethanol, about 10 seconds;
Hydrate with 90% ethanol, about 10 seconds;
Hydrate with 100% ethanol, about 10 seconds (check the sections in the microscope); xylene, about 1min.

The term "about" in the above protocol includes deviations of up to 100% (as regards time intervals). It is also envisaged that a step which is said to be carried out for example twice, is carried out three times or just one time etc - the skilled person is well aware that it is possible to amend the above protocol to some extent. It is to be understood that the above protocol merely serves as a guideline for carrying out a successful fixation procedure.

It is envisaged that the respective sections can be examined by fluorescence microscopy.

We injected 50 microgram of Cy5 labeled Herceptin, Omnitarg and Xolair in Calu3 (Her2 positive tumor) bearing mice. Herceptin and Omnitarg are therapeutic antibodies against Her2 (positive control groups) and Xolair is an antibody that binds to human IgE (negative control group). 48 hours after injection a strong fluorescence signal is detectable in the tumor area of Herceptin-Cy5 and Omnitarg-Cy5 treated mice, whereas the Xolair-Cy5 treated group showed now fluorescence signal in the tumor (Fig. 1a-c). After the in vivo imaging the tumors were explanted, fixed in formalin and afterwards dehydrated and embedded in paraffin. Paraffin-embedded tissues were cut to 2 µm slices, mounted on microscope slides and incubated over night by 39°C. Subsequent analysis of explanted tumor tissue reveals that the Her2 specific antibodies bind only to tumor cells, but not to murine tissue. In contrast, no fluorescence signal is generated with the control antibody Xolair which is directed to human IgE (Fig. 3a-3c).
To verify the specific binding of the labeled antibody to the tumor cells, we use a new developed staining procedure which makes it possible to get bright field and fluorescence information out of one tissue slide. With the normal staining procedure you need two serial tissue slides. One slide for the Hematoxylin (basophilic structures) / Eosin (eosinophilic structures) staining, which provides you a very detailed and fine structured morphological overview. The second slide is used to visualize the fluorescence signals of the Cy5 labeled antibody. The disadvantages of these normal method are the morphological differences of the two tissue slides and the additional preparation time for the fluorescence staining of the cell nucleus and the cell tissue. With our new approach you can get these information's out of one tissue slide. For this, we changed the concentration and incubation times of the existing Roche Hematoxylin / Eosin staining protocol. Hematoxylin and Eosin show an activation under fluorescence conditions so that we can visualize more or less the same morphological details like in a bright field measurement. With our special fluorescence camera system (Nuance; CRi) it is possible to separate the fluorescence spectra of different fluorophors. These multispectral imaging system enable users to quantitate molecular markers even when they are colocalized in a single tissue section, producing clear and accurate images of each individual label on a multi-label tissue section. These systems also offer us the powerful capability to unmix and remove autofluorescence in Nuance fluorescence images, thereby dramatically increasing signal-to-noise and improving the accuracy of your results. In the new approach we first measure the optimized Hematoxylin / Eosin staining of the tissue slide in the bright field (Fig. 2a-2c). Than switch the same slide area into fluorescence and measure the Hematoxylin and Eosin signals plus the Cy5 labeled antibody signal. The Nuance system separate these three fluorescence signals from the rest of the unwanted slide information's (e.g. autofluorescence) and stitch them in one image together (Fig. 3a-3c).

It is therefore envisaged that the methods of the present invention are preferably carried out as specified above. In particular, it is envisaged that the subset of cells (which is preferably comprised in a tissue sample) is stained with Hematoxylin / Eosin and subsequently analyzed with a fluorescence detection device (for example a fluorescence camera system (Nuance; CRi)) or comparable systems from Olympus, which is/are able to separate the fluorescence spectra of different fluorophors. Software etc, which might be used or designed for that purpose, is well known to the skilled person.

Once a "signal" is obtained, proving that signal truly reflects the distribution of the target is still a matter of some difficulty. The simplest negative control is the absence of expression in tissues in which the RNA for the target is known not to be expressed by RNase protection, and restriction of expression to regions of a target tissue that have expressed RNA as seen by in situ hybridization. An alternative negative control is the elimination of the signal by pre-incubating the binding domain with an excess of the peptide or protein with which it was raised. A Western blot can suggest specificity of the interaction if only one band is seen; nonetheless, the difficulty of optimizing conditions for a Western blot should demonstrate the possibility that conditions in the immunohistochemistry reaction are less than optimal.

It is envisaged that in the methods of the present invention, said detection further comprises in situ hybridization techniques, preferably fluorescent in situ hybridization (FISH).

The present invention also relates to a kit comprising a binding domain as defined herein, and means to administer said binding domain to a subject, and means to detect said binding domain with a method defined herein. Said kit may further comprise leaflets comprising instructions on how to employ the binding domain and in particular to employ the binding domain in the methods of the present invention. The kits of the present invention comprise in a preferred embodiment a binding domain as described herein, which binding domain is labeled, preferably with a fluorescent dye, and, additionally administration means like for example a syringe, a pharmaceutically acceptable carrier to dilute said binding domain etc. and, preferably, also technical instructions which aid the skilled person in the administration of the binding domain to the respective subject (it is in particular important that the binding domain is to be administered before the tissue/subset of cells is removed). The kits may further comprise means to remove a tissue sample from said subject once the binding domain has been administered to the very subject, and/or means to fix the removed tissue sample such as buffers which allow for a fixation of the removed tissue sample and/or glass slides for the subsequent fluorescence microscopy, and/or paraffin to paraffinize the removed tissue sample, and/or organic solvents to remove the paraffin etc.

The present invention also relates to an IHC method for detecting a subset of cells, with a binding domain which is specific for a target which characterizes said subset of cells, which method comprises detecting in a tissue section, in vitro, said subset of cells, wherein said binding domain has bound its target prior to the fixation of said subset of cells and/or prior to the detection of said target, wherein said binding domain is labeled and wherein said subset of cells is detected by direct immunohistochemistry.

The present disclosure also relates to a method for detecting a target which characterizes said subset of cells, with a binding domain which is specific for said target, which method comprises detecting, in vitro, said target, wherein said binding domain has bound its target prior to the fixation of said subset of cells and/or prior to the detection of said target.

The methods" for detecting a subset of cells" as defined herein may additionally or alternatively be used for:
(i) identifying a subject disposed to respond favorably to a binding domain (said subject is preferably a subject suffering from a tumor), or
(ii) monitoring a therapy, preferably a anti-cancer/tumor therapy which is based on a binding domain (i.e. the binding domain is therapeutically active), or
(iii) identifying a binding domain capable of binding in vivo to a target,
(iv) selecting a binding domain disposed to act therapeutically effective on a tumor characterized by a target;
(v) typifying a tumor.

It will be understood that the term "detecting a subset of cells" as used herein includes situations wherein not all (quantitatively) individual cells of the mentioned subset of cells is bound by the binding domain (although these cells are characterized by the target as well). Due to the natural limitations of the accumulation of binding domains in a cell mass like a tumor, it is possible and therefore envisaged that not all target cells defining the subset of cells are bound by the binding domain.

Advantageously, the methods of the present invention allow a verification of target expression and demonstration of binding of the therapeutic antibody simultaneously in vivo at the time point when therapy is initiated. The methods of the present invention may thus be applied for the stratification of patients. In the adjuvant setting, patients will be treated first with a small amount of the labelled therapeutic antibody. Subsequently tumor tissue will be surgically removed about 24 to 48 hours later, fixed in formalin and embedded in paraffin. Expression of the tumor-associated antigen and specific binding of the antibody to the tumor cells can be confirmed by near infrared based microscopy. The identification of a subject disposed to respond favourably to a binding domain may alternatively be denoted as "stratified medicine". Stratified medicine is the management of a group of patients with shared biological characteristics (e.g. a target or subset of cells as defined herein) by using molecular diagnostic testing to select the most optimal therapy in order to achieve the best possible medicinal outcome for that group. It is for example envisaged that in order to treat a certain tumor in a patient, one characterizes which binding domain (e.g. a monoclonal antibody which is or which is assumed to be therapeutically active) has the best binding characteristics for a group of patients or even for a specific patient. Thus, by way of employing the embodiments of the present invention it is possible to find the best binding domain for a patient (personalized medicine). The great advantage of the present invention is thereby, that the therapeutic active binding domains (e.g. antibodies) may also be used for the IHC based detection which leads to results reflecting the actual in vivo situation. In vivo situation means that it might be that some patients respond more favourably on antibody A, while other react more favourably to antibody B (both antibodies are thereby specific for the same target, e.g. for Her2/neu). Differences in the binding characteristics may thus guide the skilled person to employ the best possible binding domain for a given therapeutic situation (e.g. a tumor). The binding domain can thus be specifically chosen for a specific patient. It is for example envisaged to provide a set of antibodies all of which bind to the same target, and to test which of these antibodies shows the most promising binding characteristics for the respective patient (personalized medicine).

"Monitoring a therapy" includes that it is now possible to monitor the presence, absence, over-expression, under-expression of a given target or subset of cells characterized by that target with the therapeutically active antibody which already is, or which may be used for the treatment of a disease which disease is characterized by that target and/or subset. Thus, it is now for example possible to treat a tumor with a specific antibody in a patient and to monitor the "success" of the therapy with one and the same antibody. In this regard, it is envisaged that the therapeutic antibody is labelled or at least partly labelled (e.g. 10% of the total antibody population is labelled while the rest is unlabeled) and that the response in vivo is monitored from time to time. It is also envisaged that the therapeutically effective, but non-labelled antibody is replaced from time to time by the same antibody in labelled form in order to monitor the response in vivo.

"Identifying a binding domain capable of binding in vivo to a target" means that it is now possible to provide a set of binding domains, (a) which set is, or is assumed to be therapeutically effective; or (b) which set comprises or is assumed to comprise binding domains which are therapeutically effective, and to test whether these binding domains are capable of binding to the respective target, thereby indicating that said binding domain is therapeutically effective, either in general or in a subgroup of patients or even in only one patient (personalized identification). It is thus possible to select a binding domain disposed to act therapeutically effective on an tumor (which tumor is characterized by said target).

"Typifying a tumor" means to evaluate the status of a tumor, for example the HER2/neu status.

The present disclosure also relates to the use of a binding domain, preferably a therapeutically effective antibody like for example alemtuzumab, apolizumab, cetuximab, epratuzumab, galiximab, gemtuzumab, ipilimumab, labetuzumab, panitumumab, rituximab, trastuzumab, nimotuzumab, mapatumumab, matuzumab, rhMab ICR62, rhMab B-Ly1 and pertuzumab etc., including combinations thereof, for the preparation of a pharmaceutical composition for the treatment of patients disposed to respond favorably to said binding domain as identified by a method described herein.

The present disclosure also relates to a therapeutically active binding domain, preferably a therapeutically active antibody like for example alemtuzumab, apolizumab, cetuximab, epratuzumab, galiximab, gemtuzumab, ipilimumab, labetuzumab, panitumumab, rituximab, trastuzumab, nimotuzumab, mapatumumab, matuzumab, rhMab ICR62, rhMab B-Ly1 and pertuzumab etc. including combinations thereof, for use in the treatment of patients disposed to respond favorably to said binding domain as identified by a method described herein.

### The figures show:

**Figure 1 -3**: Correlation of NIRF signal intensities in vivo (Fig 1a,b,c) with formalin fixed/ paraffin embedded HE tissue slides (Fig 2a,b,c) multispectral imaging of explanted tumor tissue (Fig 3a,b,c) using labeled Herceptin (a), Omnitarg (b) and Xolair (c).

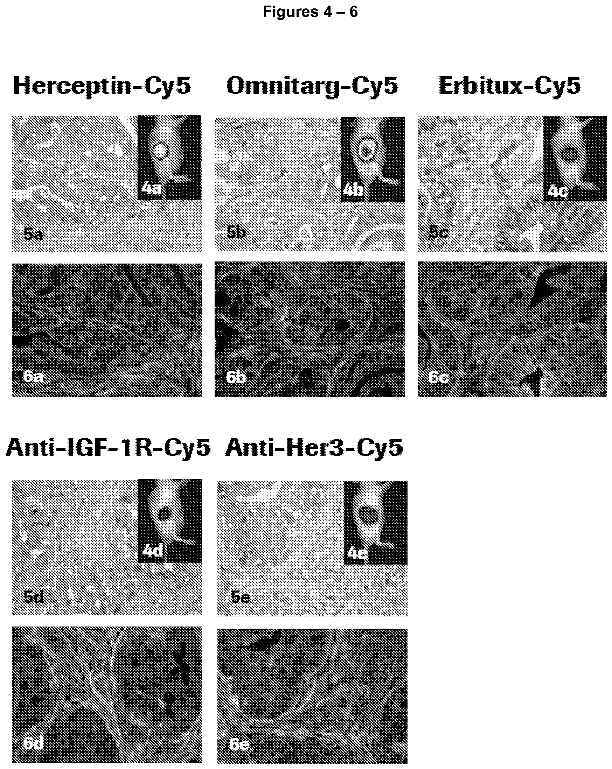

**Figure 4-6****:** Correlation of in vivo and ex vivo NIRF signal intensities. A strong in vivo fluorescence signaling could be visualized in the Her2 positive tumor with Cy5 labeled Herceptin and Omnitarg (Fig. 4a, 4b). No specific fluorescence signal could be detected with the Cy5 labeled Erbitux, anti-IGF-1R and anti-Her3 (Fig. 4c-4e). The ex vivo analysis of the explanted, formalin fixed and paraffin embedded tumors confirm these results. Specific binding of the labeled antibody could be visualized only with Herceptin-Cy5 and Omnitarg-Cy5, whereas Erbitux-Cy5, anti-IGF-1R-Cy5 and anti-Her3-Cy5 show no fluorescence signal on the tumor cells (Fig. 6a-6e).

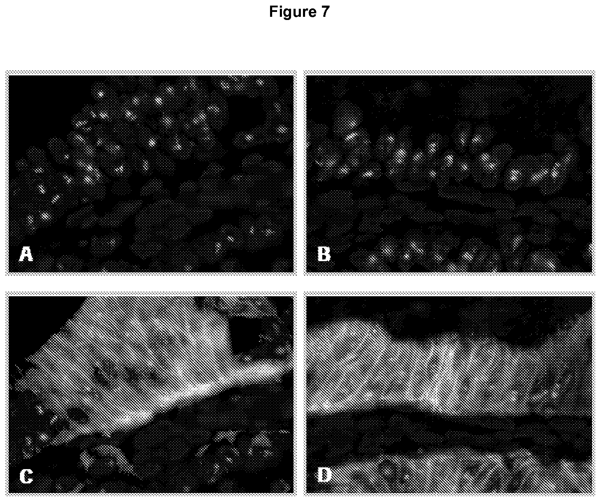

**Figure 7** Fig 7A and Fig 7B demonstrate Her2 gene expression indicated by the green colour. Cells without Her2 gene expression have been identified as murine stroma cells. Fig 7C and Fig 7D demonstrates specific binding of the Cy5 labeled Herceptin (anti-Her2 monoclonal antibody) to the Her2 gene expressing tumor cells.

This disclosure may best be understood in conjunction with the accompanying drawings. Furthermore, a better understanding of the present invention and of its many advantages will be had from the following examples, given by way of illustration and are not intended as limiting.

### Examples:

The following examples illustrate the invention. These examples should not be construed as to limit the scope of this invention. The examples are included for purposes of illustration and the present invention is limited only by the claims.

### Example 1

For the validation of these new approach we injected 50 microgram of Cy5 labeled Herceptin, Omnitarg and Xolair in Calu3 (Her2 positive tumor) bearing mice. Herceptin and Omnitarg are therapeutic antibodies against Her2 (positive control groups) and Xolair is an antibody that binds to human IgE (negative control group). 48 hours after injection a strong fluorescence signal is detectable in the tumor area of Herceptin-Cy5 and Omnitarg-Cy5 treated mice, whereas the Xolair-Cy5 treated group showed now fluorescence signal in the tumor (Fig. 1a-c). After the in vivo imaging the tumors were explanted, fixed in formalin and afterwards dehydrated and embedded in paraffin. Paraffin-embedded tissues were cut to 2 µm slices, mounted on microscope slides and incubated over night by 39°C. Subsequent analysis of explanted tumor tissue reveals that the Her2 specific antibodies bind only to tumor cells, but not to murine tissue. In contrast, no fluorescence signal is generated with the control antibody Xolair which is directed to human IgE (Fig. 3a-3c).

To verify the specific binding of the labeled antibody to the tumor cells, we use a new developed staining procedure which makes it possible to get bright field and fluorescence information out of one tissue slide. With the normal staining procedure you need two serial tissue slides. One slide for the Hematoxylin (basophilic structures) / Eosin (eosinophilic structures) staining, which provides you a very detailed and fine structured morphological overview. The second slide is used to visualize the fluorescence signals of the Cy5 labeled antibody. The disadvantages of these normal method are the morphological differences of the two tissue slides and the additional preparation time for the fluorescence staining of the cell nucleus and the cell tissue. With our new approach you can get these information's out of one tissue slide. For this, we changed the concentration and incubation times of the existing Roche Hematoxylin / Eosin staining protocol. Hematoxylin and Eosin show an activation under fluorescence conditions so that we can visualize more or less the same morphological details like in a bright field measurement. With our special fluorescence camera system (Nuance; CRi) it is possible to separate the fluorescence spectra of different fluorophors. These multispectral imaging system enable users to quantitate molecular markers even when they are colocalized in a single tissue section, producing clear and accurate images of each individual label on a multi-label tissue section. These systems also offer us the powerful capability to unmix and remove autofluorescence in Nuance fluorescence images, thereby dramatically increasing signal-to-noise and improving the accuracy of your results. In the new approach we first measure the optimized Hematoxylin / Eosin staining of the tissue slide in the bright field (Fig. 2a-2c). Than switch the same slide area into fluorescence and measure the Hematoxylin and Eosin signals plus the Cy5 labeled antibody signal. The Nuance system separate these three fluorescence signals from the rest of the unwanted slide information's (e.g. autofluorescence) and stitch them in one image together (Fig. 3a-3c).

### Example 2

We confirmed the results from the first example in a second experiment. For this, the same experimental setting was used but with some additional Cy5 labeled antibodies (Erbitux, anti-IGF-1R, anti-Her3). The strong in vivo signals form Herceptin- Cy5 and Omnitarg-Cy5 correlates with strong ex vivo signaling in the tissue slides (Fig. 4a, 4b, 6a, 6b). On the other hand Erbitux-Cy5, anti-IGF-1R-Cy5 and anti-Her3-Cy5 showed no significant in vivo and ex vivo fluorescence. The results of the in vivo and ex vivo imaging match perfectly to the previous shown data from example 1.

### Example 3

Subsequent studies are aimed to proof if the labeled antibody binds specifically to the tumor cells. Slides from Fig. 1a have been used for fluorescence in situ hybridization. With this assay the Her2 gene can be detected in formalin fixed tissue slides. Fig. 7b demonstrate that the Cy5 labeled anti-Her2 antibody (Herceptin) binds only to cells which express the Her2 gene.

## Claims

1. An immunohistochemistry (IHC) method for detecting a subset of cells with a binding domain which is specific for a target which characterizes said subset of cells, which method comprises detecting in a tissue section, ex vivo, said subset of cells, wherein said binding domain is to be administered to a subject which comprises or is assumed to comprise said subset of cells, prior to the removal of said subset of cells, wherein said binding domain is labeled and wherein said subset of cells is detected by direct immunohistochemistry.

2. The method of claim 1, wherein said subset of cells is composed of tumor cells.

3. The method of claims 1 or 2, wherein said binding domain is labeled with a fluorescent label.

4. The method of any one of the preceding claims, wherein said target is a protein, a peptide, an enzyme, a nucleic acid, a polysaccharide, a lipid, a receptor, a cellular target, and/or a tumor antigen.

5. The method of any one of the preceding claims, wherein said binding domain is an antibody.

6. The method of any one of the preceding claims, wherein said detection by direct immunohistochemistry comprises detecting the presence of said (fluorescently) labeled binding domain.

7. The method of any one of the preceding claims, wherein said subject has received said binding domain in a therapeutically effective dose prior to the removal of said tissue sample.

8. Use of a binding domain as defined in any one of the preceding claims in a method defined in any one of the preceding claims.

9. Use of a binding domain as defined in any one of the preceding claims for the preparation of a diagnostic composition to be used in a method defined in any one of the preceding claims.

10. Binding domain as defined in any one of the preceding claims to be used in a method defined in any one of the preceding claims.

11. Kit comprising a binding domain as defined in any one of the preceding claims, and means to administer said binding domain to a subject, and means to detect said binding domain with a method defined in any one of the preceding claims.

12. The method as defined in any one of the preceding claims for:
(i) identifying a subject disposed to respond favorably to a therapeutically active binding domain, or
(ii) monitoring a therapy, or
(iii) identifying a binding domain capable of binding to a target in vivo, or
(iv) selecting an binding domain disposed to act therapeutically active on an tumor **characterized by** a target; or
(v) typifying a tumor.

## Patentansprüche

1. Immunhistochemisches (IHC)-Verfahren für den Nachweis einer Untergruppe von Zellen mit einer Bindungsdomäne, die für ein Ziel spezifisch ist, das die Untergruppe von Zellen charakterisiert, wobei das Verfahren den Nachweis der Untergruppe von Zellen in einem Gewebeschnitt ex vivo umfasst, wobei die Bindungsdomäne einem Patienten, der die Untergruppe von Zellen umfasst oder von dem angenommen wird, dass er sie umfasst, vor dem Entfernen der Untergruppe von Zellen zu verabreichen ist, wobei die Bindungsdomäne markiert ist und wobei die Untergruppe von Zellen durch direkte Immunhistochemie nachgewiesen wird.

2. Verfahren nach Anspruch 1, wobei die Untergruppe von Zellen aus Tumorzellen besteht.

3. Verfahren nach Anspruch 1 oder 2, wobei die Bindungsdomäne mit einer Fluoreszenzmarkierung markiert ist.

4. Verfahren nach einem der vorhergehenden Ansprüche, wobei das Ziel ein Protein, ein Peptid, ein Enzym, eine Nukleinsäure, ein Polysaccharid, ein Lipid, ein Rezeptor, ein zelluläres Ziel und/oder ein Tumor-antigen ist.

5. Verfahren nach einem der vorhergehenden Ansprüche, wobei die Bindungsdomäne ein Antikörper ist.

6. Verfahren nach einem der vorhergehenden Ansprüche, wobei der Nachweis durch direkte Immunhistochemie den Nachweis des Vorhandenseins der (fluoreszierend) markierten Bindungsdomäne umfasst.

7. Verfahren nach einem der vorhergehenden Ansprüche, wobei der Patient die Bindungsdomäne vor dem Entfernen der Gewebeprobe in einer therapeutisch wirksamen Dosis erhalten hat.

8. Verwendung einer Bindungsdomäne nach einem der vorhergehenden Ansprüche in einem Verfahren nach einem der vorhergehenden Ansprüche.

9. Verwendung einer Bindungsdomäne nach einem der vorhergehenden Ansprüche für die Herstellung einer diagnostischen Zusammensetzung, die in einem Verfahren nach einem der vorhergehenden Ansprüche zu verwenden ist.

10. Bindungsdomäne nach einem der vorhergehenden Ansprüche, die in einem Verfahren nach einem der vorhergehenden Ansprüche zu verwenden ist.

11. Kit, umfassend eine Bindungsdomäne nach einem der vorhergehenden Ansprüche, und Mittel, um die Bindungsdomäne an einen Patienten zu verabreichen, und Mittel, um die Bindungsdomäne mit einem Verfahren nach einem der vorhergehenden Ansprüche nachzuweisen.

12. Verfahren nach einem der vorhergehenden Ansprüche, um:
(i) einen Patienten zu ermitteln, der so disponiert ist, dass er positiv auf eine therapeutisch wirksame Bindungsdomäne anspricht, oder
(ii) eine Therapie zu überwachen, oder
(iii) eine Bindungsdomäne zu ermitteln, die sich in vivo an ein Ziel binden kann, oder
(iv) eine Bindungsdomäne auszuwählen, die so disponiert ist, dass sie auf einen durch ein Ziel charakterisierten Tumor therapeutisch wirken kann, oder
(v) einen Tumor zu typisieren.

## Revendications

1. Procédé d'immunohistochimie (IHC) pour détecter un sous-ensemble de cellules avec un domaine de liaison qui est spécifique à une cible qui caractérise ledit sous-ensemble de cellules, lequel procédé consistant à détecter, dans une section de tissu, ex vivo, ledit sous-ensemble de cellules, dans lequel ledit domaine de liaison doit être administré à un sujet qui comprend ou est supposé comprendre ledit sous-ensemble de cellules, avant l'élimination dudit sous-ensemble de cellules,
dans lequel ledit domaine de liaison est marqué et dans lequel ledit sous-ensemble de cellules est détecté par immunohistochimie directe.

2. Procédé selon la revendication 1, dans lequel ledit sous-ensemble de cellules est composé de cellules tumorales.

3. Procédé selon les revendications 1 ou 2, dans lequel ledit domaine de liaison est marqué avec une étiquette fluorescente.

4. Procédé selon l'une quelconque des revendications précédentes, dans lequel ladite cible est une protéine, un peptide, une enzyme, un acide nucléique, un polysaccharide, un lipide, un récepteur, une cible et/ou un antigène tumoral.

5. Procédé selon l'une quelconque des revendications précédentes, dans lequel ledit domaine de liaison est un anticorps.

6. Procédé selon l'une quelconque des revendications précédentes, dans lequel ladite détection par direct immunohistochimie comprend la détection de la présence dudit domaine de liaison marqué (par fluorescence).

7. Procédé selon l'une quelconque des revendications précédentes, dans lequel ledit sujet a reçu ledit domaine de liaison en une dose thérapeutiquement efficace avant l'élimination dudit échantillon de tissu.

8. Utilisation d'un domaine de liaison tel que défini dans l'une quelconque des revendications précédentes dans un procédé défini dans l'une quelconque des revendications précédentes.

9. Utilisation d'un domaine de liaison tel que défini dans l'une quelconque des revendications précédentes pour la préparation d'une composition de diagnostic à utiliser dans un procédé défini dans l'une quelconque des revendications précédentes.

10. Domaine de liaison tel que défini dans l'une quelconque des revendications précédentes à utiliser dans un procédé défini dans l'une quelconque des revendications précédentes.

11. Kit comprenant un domaine de liaison tel que défini dans l'une quelconque des revendications précédentes, et des moyens pour administrer ledit domaine de liaison à un sujet, et des moyens pour détecter ledit domaine de liaison avec un procédé défini dans l'une quelconque des revendications précédentes.

12. Procédé selon l'une quelconque des revendications précédentes pour:
(i) identifier un sujet disposé à répondre favorablement à un domaine de liaison thérapeutiquement actif, ou
(ii) surveiller une thérapie, ou
(iii) identifier un domaine de liaison capable de se lier à une cible in vivo, ou
(iv) sélectionner un domaine de liaison disposé à agir de manière thérapeutiquement active sur une tumeur **caractérisée par** une cible; ou
(v) typifier une tumeur.
